(19) Europäisches Patentamt
European Patent Office
Office européen des brevets

(11) Veröffentlichungsnummer: **0 489 323 A1**

(12) # EUROPÄISCHE PATENTANMELDUNG

(21) Anmeldenummer: **91120028.5**

(22) Anmeldetag: **25.11.91**

(51) Int. Cl.5: **C07D 339/06**, C07D 339/08, C12P 41/00, C12P 13/04

(30) Priorität: **06.12.90 DE 4038834**

(43) Veröffentlichungstag der Anmeldung: **10.06.92 Patentblatt 92/24**

(84) Benannte Vertragsstaaten: **AT BE CH DE DK ES FR GB GR IT LI LU NL SE**

(71) Anmelder: **BAYER AG**

**W-5090 Leverkusen 1 Bayerwerk(DE)**

(72) Erfinder: **Bender, Wolfgang, Dr.**
**Claudiusweg 5**
**W-5600 Wuppertal(DE)**
Erfinder: **Schutt, Hermann, Dr.**
**Gellertweg 12**
**W-5600 Wuppertal(DE)**

(54) **Optisch reine Dithiolanyl- und Dithianoaminosäuren und deren Derivate, Verfahren zu ihrer Herstellung und ihre Verwendung.**

(57) Die vorliegende Erfindung betrifft optisch reine S- und R-Dithiolanyl- und Dithianoaminosäuren und deren Derivate der allgemeinen Formel (I)

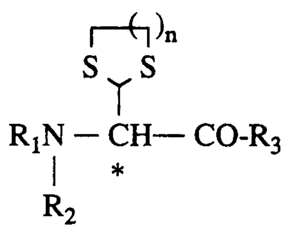

(I)

in welcher n, $R^1$, $R^2$ und $R^3$ die in der Beschreibung angegebene Bedeutung haben, ein enzymatisches Verfahren zu ihrer Herstellung durch stereoselektive Spaltung der entsprechenden racemischen R,S-Verbindungen, sowie ihre Verwendung als Zwischenprodukte für die Herstellung von Arzneimitteln.

EP 0 489 323 A1

Die vorliegende Erfindung betrifft optisch reine S- und R-Dithiolanyl- und Dithianoaminosäuren und deren Derivate, ein enzymatisches Verfahren zu ihrer Herstellung durch stereoselektive Spaltung der entsprechenden racemischen R,S-Verbindungen, sowie ihre Verwendung als Zwischenprodukte für die Herstellung von Arzneimitteln.

Optisch reine heterocyclische Aminosäuren und ein enzymatisches Verfahren zu ihrer Herstellung sind aus US P 4 389 489 bereits bekannt.

Die Erfindung betrifft neue optisch reine Verbindungen der allgemeinen Formel (I)

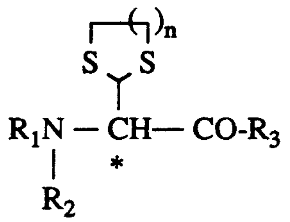

(I)

in welcher

| | |
|---|---|
| n | für die Zahl 1 oder 2 steht, |
| $R^1$ | für Wasserstoff, geradkettiges oder verzweigtes Alkyl mit bis zu 8 Kohlenstoffatomen, Benzyl oder für eine Gruppe der Formel $-COR^4$ steht, worin |
| $R^4$ | geradkettiges oder verzweigtes Alkyl mit bis zu 10 Kohlenstoffatomen oder Aryl mit 6 bis 10 Kohlenstoffatomen bedeutet, oder |
| | für eine typische Aminoschutzgruppe steht, |
| $R^2$ | für Wasserstoff, geradkettiges oder verzweigtes Alkyl mit bis zu 8 Kohlenstoffatomen oder Benzyl steht, oder |
| $R^1$ und $R^2$ | gemeinsam mit dem Stickstoffatom einen Morpholinring oder einen Phthalimidorest bilden, |
| $R^3$ | für Hydroxy, geradkettiges oder verzweigtes, gegebenenfalls durch Vinyl substituiertes Alkoxy mit bis zu 10 Kohlenstoffatomen, Benzyloxy oder für die Gruppe $-NR^5R^6$ steht, worin |
| | $R^5$ und $R^6$ gleich oder verschieden sind und Wasserstoff, geradkettiges oder verzweigtes Alkyl mit bis zu 6 Kohlenstoffatomen oder Phenyl bedeuten, |

und das mit * gekennzeichnete C-Atom entweder in der S- oder R-Form vorliegt.

Aminoschutzgruppe im Rahmen der Erfindung sind die üblichen in der Peptid-Chemie verwendeten Aminoschutzgruppen.

Hierzu gehören bevorzugt: Benzyloxycarbonyl, 3,4-Dimethoxybenzyloxycarbonyl, 3,5-Dimethoxybenzyloxycarbonyl, 2,4-Dimethoxybenzyloxycarbonyl, 4-Methoxybenzyloxycarbonyl, 4-Nitrobenzyloxycarbonyl, 2-Nitrobenzyloxycarbonyl, 2-Nitro-4,5-dimethoxybenzyloxycarbonyl, Methoxycarbonyl, Ethoxycarbonyl, Propoxycarbonyl, Isopropoxycarbonyl, Butoxycarbonyl, Isobutoxycarbonyl, tert.-Butoxycarbonyl, Allyloxycarbonyl, Vinyloxycarbonyl, 2-Nitrobenzyloxycarbonyl, 3,4,5-Trimethoxybenzyloxycarbonyl, Cyclohexoxycarbonyl, 1,1-Dimethylethoxycarbonyl, Adamantylcarbonyl, Phthaloyl, 2,2,2-Trichlorethoxycarbonyl, 2,2,2-Trichlor-tertbutoxycarbonyl, Menthyloxycarbonyl, Phenoxycarbonyl, 4-Nitrophenoxycarbonyl, Fluorenyl-9-methoxycarbonyl, Formyl, Acetyl, Propionyl, Pivaloyl, 2-Chloracetyl, 2-Bromacetyl, 2,2,2-Trifluoracetyl, 2,2,2-Trichloracetyl, Benzoyl, 4-Chlorbenzoyl, 4-Brombenzoyl, 4-Nitrobenzoyl, Phthalimido, Isovaleroyl oder Benzyloxymethylen, 4-Nitrobenzyl, 2,4-Dinitrobenzyl, 4-Nitrophenyl.

Bevorzugt sind Verbindungen der allgemeinen Formel (I), in welcher

| | |
|---|---|
| n | für eine Zahl 1 oder 2 steht, |
| $R^1$ | für Wasserstoff, geradkettiges oder verzweigtes Alkyl mit bis zu 6 Kohlenstoffatomen oder Benzyl steht, oder für eine Gruppe der Formel $-COR^4$ steht, worin |
| $R^4$ | geradkettiges oder verzweigtes Alkyl mit bis zu 8 Kohlenstoffatomen oder Phenyl bedeutet, oder |
| | für tert.Butoxycarbonyl (Boc), Benzyloxycarbonyl (Z), Ethoxycarbonyl (EtOC), Allyloxycarbonyl (AllOC), Fluorenyl-9-methoxycarbonyl (Fmoc) oder Methoxycarbonyl (MetOC) steht, |
| $R^2$ | für Wasserstoff, geradkettiges oder verzweigtes Alkyl mit bis zu 6 Kohlenstoffatomen oder Benzyl steht, oder |
| $R^1$ und $R^2$ | gemeinsam mit dem Stickstoffatom den Phthalimidorest bilden, |

$R^3$ für Hydroxy, geradkettiges oder verzweigtes, gegebenenfalls durch Vinyl substituiertes Alkoxy mit bis zu 8 Kohlenstoffatomen, Benzyloxy oder für die Gruppe $-NR^5R^6$ steht, worin

$R^5$ und $R^6$ gleich oder verschieden sind und Wasserstoff, geradkettiges oder verzweigtes Alkyl mit bis zu 4 Kohlenstoffatomen oder Phenyl bedeuten,

und das mit * gekennzeichnete C-Atom entweder in der S- oder R-Form vorliegt.

Besonders bevorzugt sind Verbindungen der allgemeinen Formel (I), in welcher

n für die Zahl 1 oder 2 steht,

$R^1$ für Wasserstoff, geradkettiges oder verzweigtes Alkyl mit bis zu 4 Kohlenstoffatomen oder Benzyl steht, oder

für eine Gruppe der Formel $-CO-R^4$ steht,

worin

$R^4$ geradkettiges oder verzweigtes Alkyl mit bis zu 8 Kohlenstoffatomen oder Phenyl bedeutet, oder

für tert.-Butoxycarbonyl (BOC), Benzyloxycarbonyl(Z), Allyloxycarbonyl (AllOC), Ethoxycarbonyl (EtOC) oder Fluorenyl-9-methoxycarbonyl (Fmoc) steht,

$R^2$ für Wasserstoff, geradkettiges oder verzweigtes Alkyl mit bis zu 4 Kohlenstoffatomen oder Benzyl steht, oder

$R^1$ und $R^2$ gemeinsam mit dem Stickstoffatom den Phthalimidorest bilden,

$R^3$ für Hydroxy, geradkettiges oder verzweigtes, gegebenenfalls durch Vinyl substituiertes Alkoxy mit bis zu 8 Kohlenstoffatomen, Benzyloxy oder für die Gruppe $-NR^5R^6$ steht, worin

$R^5$ und $R^6$ gleich oder verschieden sind und Wasserstoff, Methyl, Ethyl, Propyl, Isopropyl oder Phenyl bedeuten

und das mit * gekennzeichnete C-Atom entweder in der S- oder R-Form vorliegt.

Außerdem wurde in Verfahren zur Herstellung der erfindungsgemäßen optisch reinen Verbindungen der allgemeinen Formel (I) gefunden, dadurch gekennzeichnet, daß man die racemischen Verbindungen der allgemeinen Formel (II)

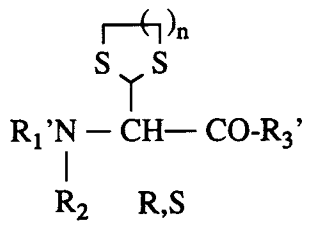

(II)

in welcher

n und $R^2$ die oben angegebene Bedeutung haben,

$R^{1'}$ und $R^{3'}$ die oben angegebenen Bedeutungen von $R^1$ und $R^3$ haben und mit diesen gleich oder verschieden sind, aber $R^{1'}$ nicht für Wasserstoff und $R^{3'}$ nicht für Hydroxy stehen darf,

unter Einwirkung von spezifisch spaltenden trägergebundenen und/oder freien proteolytischen Enzymen, in einem zweiphasigen Medium, bestehend aus Wasser und einem mit Wasser nicht mischbaren Lösemittel, in die optisch reinen R-Verbindungen der Formeln (III) oder (IV)

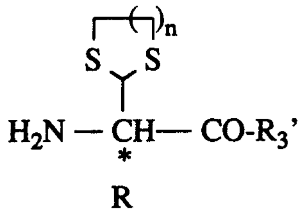 (III) oder

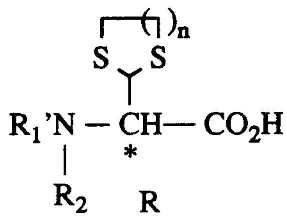 (IV)

in welchen
n, $R^1$, $R^{1'}$, $R^2$ und $R^{3'}$ die oben angegebene Bedeutung haben,
überführt, von den ebenfalls anfallenden optisch reinen S-Verbindungen der Formel (IIa)

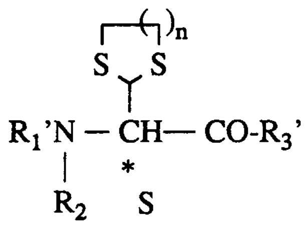 (IIa)

in welcher
$R^{1'}$,$R^2$ und $R^{3'}$ die oben angegebene Bedeutung haben,
nach üblicher Methode abtrennt, wobei diese nach üblicher chemischer Racemisierung dem enzymatischen Prozeß erneut zugeführt werden können, und
in einem letzten Schritt gegebenenfalls die Substituenten $R^{1'}$ und/oder $R^{3'}$ in den Verbindungen der allgemeinen Formeln (IIa), (III) und (IV) nach üblicher chemischer Methode in die Substituenten $R^1$ und $R^3$ überführt.

Das erfindungsgemäße Verfahren kann durch folgendes Formelschema beispielhaft erläutert werden:

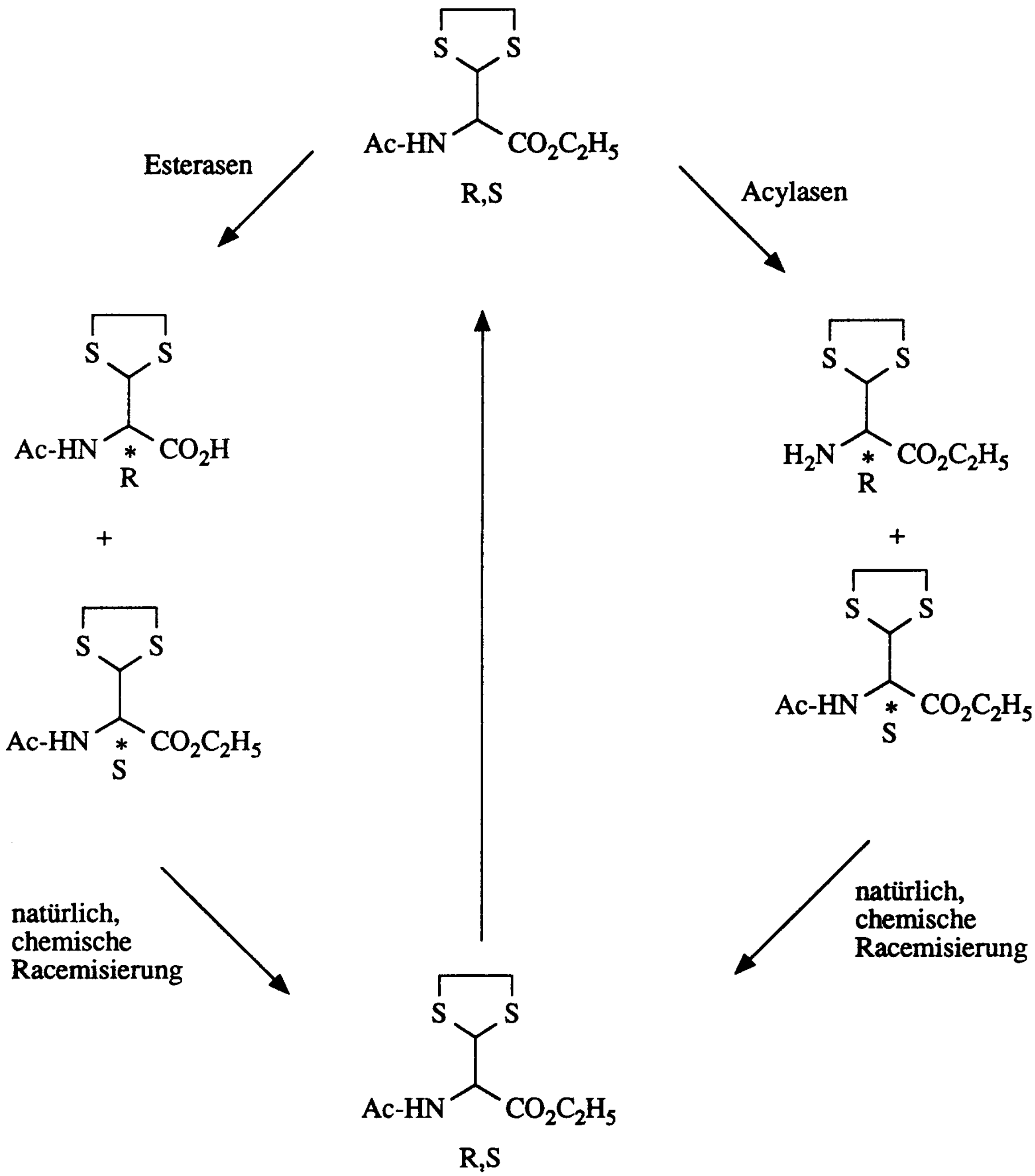


Bei dem erfindungsgemäßen Verfahren ist neben der Auswahl des Enzyms auch der Hydrolyseschritt von Bedeutung. Wie oben gezeigt, werden in den R-Verbindungen die jeweiligen Ester- bzw. Amidgruppen durch Hydrolyse in die entsprechende freie Carboxyl- oder Amingruppe überführt, während die S-Verbindungen dieser Hydrolyse nicht unterliegt. Soll die S-Form optisch rein erhalten werden, so ist auf die vollständige Hydrolyse des R-Derivates zu achten; soll hingegen die R-Form rein isoliert werden, so sollte die Hydrolyse der R-Ester und Amide nicht vollständig durchgeführt werden.

Als Lösemittel für das erfindungsgemaße Verfahren eignen sich organische, mit Wasser nicht vollständig mischbare Lösemittel wie beispielsweise Methylenchlorid, Chloroform, Toluol, Benzol, Essigsäureethylester, Petrolether, Methylisobutylketon oder Isobutanol. Bevorzugt ist Methylisobutylketon.

Die enzymatische Spaltung erfolgt in einem Temperaturbereich von 20 ° C bis 40 ° C, vorzugsweise bei 25 ° C bis 37 ° C.

Sie verläuft in einem pH-Bereich von 6 bis 8, wobei der pH-Wen vorzugsweise durch Zugabe einer starken Base bei 7,0 konstant gehalten wird.

Die racemischen Verbindungen werden als ca. 10-20%ige organische Losung zu dem im Wasser suspendierten Enzym oder Enzym-Träger zugesetzt, wobei der Lösemittelanteil bezogen auf das Gesamtvolumen maximal 75-80 Vol-% betragen kann.

Das Reaktionsmedium wird während der enzymatischen Umsetzung intensiv gerührt. Der Verlauf und der Endpunkt der enzymatischen Reaktion kann durch die Neutralisation der entstehenden $H^+$-Ionen bestimmt werden. Die Neutralisation erfolgt im allgemeinen mit organischen oder anorganischen Basen.

Als Basen eignen sich anorganische Basen wie beispielsweise Ammoniak oder Natronlauge oder organische Basen wie beispielsweise Triethylamin, Ethanolamin, Pyridin, Diisopropylethylamin oder Dimethylamin.

Bevorzugt sind Ammoniak und Natronlauge.

Als selektive proteolytische Enzyme eignen sich für das Verfahren Carboxylester- und Acylgruppen spaltbare Enzyme.

Zu den Carboxylester spaltenden Enzymen gehören Serin- und Sulfhydryl-Proteasen, wie Subtilisin, das aus Bacillus subtilis oder aus Bacillus licheniformis isoliert wird, das aus Rinder- oder Schweinepankreas isolierte α-Chymotrypsin oder TLCK-Chymotrypsin, Papain, Ficin, Bromelain oder Lipasen, das beispielsweise aus den Mikroorganismen Pseudomonas fluorescens, Mucor miehei oder Rhizopus japonicus isoliert werden kann.

Bevorzugt sind die aus Bacillus subtilis und Bacillus licheniformis isolierten proteolytischen Enzyme. Diese technischen Enzyme sind unter den Firmennamen Maxatase® (Hersteller: Gist-Brocades N.V., Delft/Niederlande), Optimase® (Hersteller: Miles-Kali Chemie, Hannover) und Alcalase® (Hersteller: Novo Industrie AS, Kopenhagen/Dänemark) bekannt.

Als Acylgruppen spaltende Enzyme eignen sich Acylase, die aus Schweinenieren oder aus Mikroorganismen wie beispielsweise Aspergillus oryzae isoliert werden.

Die proteolytischen Enzyme können durch eine kovalente Bindung über einen zur Katalyse nicht essentiellen Lysinrest an den polymeren Träger gekuppelt werden. Die durch kovalente Bindung des Enzyms an den Träger entstehenden trägergebundenen Enzyme verlieren nur sehr langsam nach vielfacher Wiederverwendung ihre Aktivität. Eine weitere Möglichkeit ist, die Adsorption des Enzyms an die Poren eines geladenen Trägers sowie die anschließende Quervernetzung mit Glutardialdehyd.

Als Enzymträger kommen polymere, poröse Träger wie Cellulosen, z.B. DEAE- oder CM-Cellulose, modifizierte Polyacrylamidgele mit Amino- oder Hydroxylgruppen oder organische Copolymerisate aus Acrylamid, Methacrylaten oder Methacrylamid und Maleinsäureandydrid nach den DE.OS 22 15 539 und 22 15 687 in Frage.

Das Enzym wird zur Kupplung mit dem polymeren Träger unter optimalen Bedingungen für die Stabilität des Enzyms zur Reaktion gebracht. Die Effektivität der Kupplung kann durch Messung der enzymatischen Aktivität am Polymer und im Waschwasser bestimmt werden. Das polymere Enzym kann bei Verwendung im Batch-Verfahren leicht durch Sedimentation oder Filtration von der Reaktionslösung abgetrennt und mehrfach eingesetzt werden. Der Enzymträger kann auch in Säulen gefüllt und in Gegenwart eines Puffersystems von Substratlösung durchströmt werden.

Vorraussetzung zur Eignung eines Enzymträgers für das erfindungsgemäße Verfahren ist ein vorheriger Test zur Adsorption der Ausgangs- und Endprodukte. Geeignete Träger dürfen die Ausgangs- und Endprodukte nur in ganz geringem Umfang oder gar nicht adsorbieren.

Als besonders gute Träger für die proteolytischen Enzyme haben sich die verschiedenen Cellulosen, derivatisierten Cellulosen, Amino- oder Hydroxylgruppen-haltigen Polyacrylamidgele sowie die durch Amino- oder Hydroxylgruppen modifizierten Anhydridharze erwiesen. Generell können als Träger alle Amino- und Hydroxylgruppen tragenden polymeren Träger eingesetzt werden, die als Ausgangs- und Endprodukte des erfindungsgemäßen Verfahrens nicht adsorbieren. Der polymere Träger wird nach an sich bekannten Methoden mit Cyanurchlorid [vgl. GB-PS 1 302 706, N.L. Smith und H.M. Lehnhoff, Arch. Biochem., 61, (1974), 392-415; T.H. Finlay et al., Arch. Biochem. 87, (1978) 77-90] oder verschiedenen Halogenpyrimidinen nach DE-OS 26 19 521 oder DE-OS 26 19 451 aktiviert.

Die racemischen Verbindungen der allgemeinen Formel (II) sind teilweise bekannt und können hergestellt werden, indem man die Verbindungen der allgemeinen Formel (V)

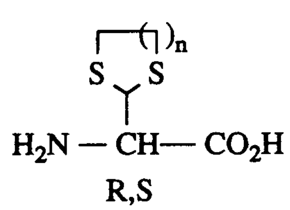

(V)

in welcher
n die oben angegebene Bedeutung hat,
im Fall des Substituenten $R^{1'}$ mit acylierenden Reagentien, beispielsweise $(R^{1'}\text{-O-CO})_2O$, $R^{1'}$-O-CO-Cl oder ($R^{1'}$-O-CO)-O-N-succinimid nach üblicher Methode in inerten Lösemitteln in Anwesenheit einer Base, beispielsweise im Gemisch Dioxan/Wasser und Natriumhydroxid oder in Dioxan mit Triethylamin in einem Temperaturbereich von 0°C bis +50°C, bevorzugt bei Raumtemperatur und Normaldruck umsetzt, und im Fall der Ester die Säuren nach literaturbekannter Methode mit den entsprechenden Alkoholen($R^{3'}$-OH) umsetzt.

Die Verbindung der Formel (V) ist bekannt [vgl. M.P. Mertes, A.A. Ramsey, J. Med. Chem. 12, 342-(1969)].

Die enantiomerenreinen Verbindungen der allgemeinen Formeln (IIa), (III) und (IV) sind neu und können nach der oben aufgeführten Methode hergestellt werden.

Die oben aufgeführte Derivatisierung nach Trennung der racemischen in die optisch reinen Verbindungen erfolgt im allgemeinen nach dem Herstellungsverfahren der Verbindungen der allgemeinen Formel (III), d.h. durch literaturbekannte Acylierung oder Veresterung [vgl. Houben-Weyl "Methoden der organischen Chemie, Synthese von Peptiden, Teil 1, 1974, Georg Thieme Verlag, Stuttgart; Th.W. Greene, Protective Groups in Organic Synthesis, John Wiley & Sons, New York].

Die Herstellung der erfindungsgemäßen, enantiomerenreinen Verbindungen kann gegebenenfalls auch über Diastereomere (beispielsweise Salze) nach üblichen Methoden wie beispielsweise fraktionierte Kristallisation, durch Säurenchromatographie oder durch Craig-Verteilung erfolgen. Welches das optimale Verfahren ist, muß von Fall zu Fall entschieden werden, manchmal ist es auch zweckmäßig, Kombinationen der einzelnen Verfahren zu benutzen. Besonders geeignet ist die Trennung durch Kristallisaiton oder Craig-Verteilung bzw. eine Kombination beider Verfahren.

Die optisch reinen Verbindungen der allgemeinen Formel (I) werden zur Synthese von pharmazeutischen Wirkstoffen, insbesondere zur Synthese von renininhibitorischen Peptiden benötigt.

Erklärungen zum experimentellen Teil:

DC-Systeme:

Stationäre Phase

Merck DC-Fertigplatten Kieselgel 60 F-254, 5 x 10cm, Schichtdicke 0,25 mm, Art.-Nr. 5719.

| Mobile Phasen (im Test als "DC-System") | | |
|---|---|---|
| I | $CH_2Cl_2$/MeOH | 9:1 |
| II | $CH_2Cl_2$/MeOH | 95:5 |
| III | $NH_3/CH_2Cl_2$/MeOH | 0,2:9:1 |
| IV | HOAc/$CH_2Cl_2$/MeOH | 0,2:9:1 |
| V | Eisessig/n-Butanol/$H_2O$ | 1:3:1 |
| VI | EtOAc/n-Hexan | 2:1 |
| VII | EtOAc/n-Hexan | 1:3 |
| VIII | EtOAc/n-Hexan | 1:1 |
| IX | $CH_2Cl_2$/MeOH | 98:2 |
| X | $CH_2Cl_2$/MeOH | 7:3 |
| XI | $CH_2Cl_2$ | 100 |

HPLC-Systeme:

HPLC-System I: Säule Merck Lichrosorb[R] RP-8, 250-4, 10 µm, Kat.-No. 50318
HPLC-System II: Säule Merck Lichrosorb[R] RP-18, 250-4, 10 µm, Kat.-No. 50334

Bestimmung der Enantiomerenverhältnisse durch Gaschromatographie an chiralen Phasen [vgl. H. Frank, g.J. Nicholson and E. Bayer, J.Chromatogr. Sci. 15 (1977), 174; H. Frank, G.J. Nicholson and E. Bayer,

Angew. Chem. 90, (1980) 396; Angew. Chem. Int Ed. Engl. 17 (1978), 363].

Abkürzungen:

| | |
|---|---|
| MIBK: | Methylisobutylketon |
| dem. Wasser: | demineralisiertes Wasser |

Ausgangsverbindungen (Racemate)

Beispiel I

N-tert.Butoxycarbonyl-2-R,S-amino-2-[2-(1,3-dithiolano)]essigsäureethylester

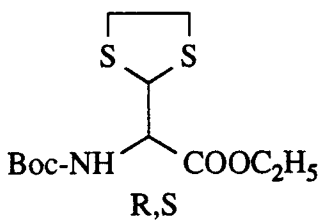

1,038 g (5 mmol) 2-R,S-Amino-2-[2-(1,3-dithiolano)]essigsäureethylester [M.P. Mertes, A.A. Ramsey, J. Med. Chem. 12, 342 (1969)] werden in 10 ml Dioxan gelöst, mit 1,038 ml (7,5 mmol) Triethylamin und schließlich unter Eiskühlung mit 1,64 g (7,5 mmol) Di-tert.Butylcarbonat versetzt. Der Ansatz wird über Nacht bei Raumtemperatur gerührt und vom Dioxan abrotiert. Der Rückstand wird in Wasser aufgenommen und 3 mal mit Diethylether ausgeschüttelt. Die vereinigte organische Phase wird 3 mal mit kalter 1 N Salzsäure und 1 mal mit Natriumbicarbonatlösung gewaschen, über Natriumsulfat getrocknet und eingeengt. Das Rohprodukt wird durch Flashchromatographie an Kieselgel mit Dichlormethan als Eluens gereinigt. Ausbeute: 1,27 g(82,6% der Theorie)
Fp.: 62 °C
DC-System I:$R_f$ = 0,89
MS-DCI: m/z = 308 (M + H); m/z = 352 ; m/z = 208
In Analogie zur Vorschrift des Beispiels I werden die in Tabelle 1 aufgeführten Beispiele hergestellt:

Tabelle 1

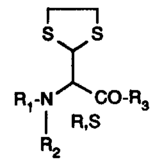

| Bsp.-Nr. | $R^1$ | $R^2$ | n | $R^3$ | F (°C) | Ausbeute (% Theorie) | DC-System ($R_f$) | HPLC ($R_t$ min) | a)MC-DCI (m/z) b) (+)FAB-MS |
|---|---|---|---|---|---|---|---|---|---|
| II | $-CO-CH_3$ | H | 1 | $-OC_2H_5$ | 107-108 | | I 0,75<br>II 0,67 | 1,73 | a) 250 (M+H) |
| III | $-CO-CH_3$ | H | 1 | -OH | | 49 | IV 0,34 | | a) 222 (M+H) |
| IV | 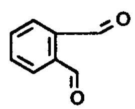 | | 2 | $-OC_2H_5$ | | 70 | II 0,89<br>XI 0,44 | II 6,63 | a) 352 (M+H)<br>204<br>119 |
| V | H | H | 2 | $-OC_2H_5$ | | 64 | II 0,80 | | b) 222 (M+H)<br>228 (M+Li)<br>224 (M+Na) |
| VI | $-CO-CH_3$ | H | 2 | $-OC_2H_5$ | | 87,8 | IV 0,76<br>V 0,89 | II 1,78 | a) 264 (M+H) |

Herstellungsbeispiele (allgemeine Formel (I))

Beispiel 1 und 2

N-Acetyl-2-S-amino-2-[2-(1,3-dithiolano)]essigsäureethylester (Beispiel 1) und N-Acetyl-2-R-amino-[2-(1,3-dithiolano)]essigsäure (Beispiel 2)

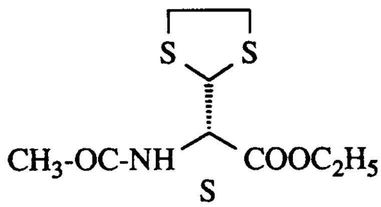

(Beispiel 1)

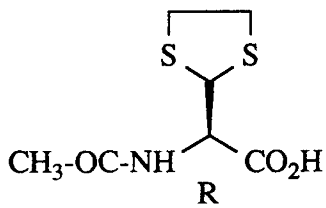

(Beispiel 2)

a) Enzymatische Spaltung

130 g (0,52 mol) der Verbindung aus dem Beispiel II werden in 1,3 l Wasser und 1,3 l MIBK (Methylisobutylketon) bei 37°C und pH 7,8 gelöst und mit dem gelösten Anteil von 26 g Maxatase puur (Gist-Brocades NV, Delft, Niederlande) in 250 ml Wasser versetzt. Der pH-Wert wird mit 2 N NaOH nachgestellt. Nach 2,5 Stunden ist die Reaktion beendet, die Phasen werden getrennt.

b) Isolierung des Beispiels 1 (N-Acetyl-S-Ester)

Die Wasserphase wird mit 2 mal 1 l MIBK nachextrahiert, die organischen Phasen werden vereinigt und am Rotationsverdampfer zur Trockene eingeengt.
Ausbeute: 64,8 g (99,6% der Theorie)

c) Isolierung des Beispiels 2 (N-Acetyl-R-Säure)

Die Wasserphase wird mit 18%iger Salzsäure auf pH 1,0 eingestellt und dort mit 3 mal 1 l MIKB extrahiert. Die organischen Phasen werden vereinigt und ebenfalls zur Trockene eingeengt.
Ausbeute: 39.9 g (67,9% der Theorie)
Analytische Daten der Beispiele 1 und 2

Beispiel 1

Fp.: 119°C
$\alpha$ $[D]_{589}$ = + 43,0; c = 1 in Methanol
DC-System I: $R_f$ = 0,75
MS-DCI: m/z = 250 (M+H)
$^1$H-NMR (DMSO, 250 MHz): $\delta$ = 1,19 (t, 3H); 1,86 (s, 3H); 3,20 (m, 4H); 4,11 (q, 2H); 4,46 (dd, 1H); 4,78 (d, 1H); 8,45 (d, 1H).
Chirale Reinheit (GC): 1,2 % R/98,8% S

Beispiel 2

$\alpha$ $[D]_{589}$ = -21,7; c = 1 in Methanol
DC-System IV: $R_f$ = 0,34
MS-DCI: m/z = 222 (M+H)
$^1$H-NMR (DMSO, 250 MHz): $\delta$ = 1,84 (s, 3H); 3,18 (m, 4H); 4,44 (dd, 1H); 4,79 (d, 1H); 8,31 (d, 1H); 12,84-

(breit, 1H).
Chirale Reinheit (GC): 93,6% R/6,5% S

Beispiele 3 und 4

N-Acetyl-2-S-amino-2-[2-(1,3-dithiano)]essigsäureethylester (Beipsiel 3) und N-Acetyl-2-R-amino-2-[2-(1,3-dithiano)]essigsäure (Beispiel 4)

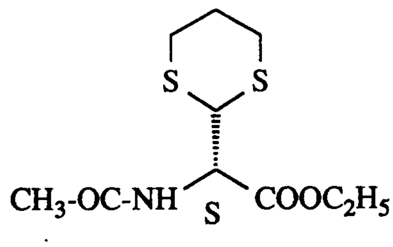

(Beispiel 3)

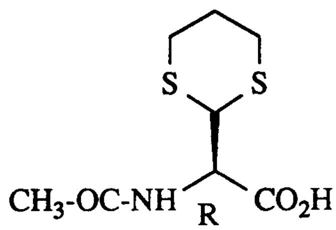

(Beispiel 4)

a) Enzymatische Spaltung des Beispiels VI

Analog der Vorschrift für die Beispiele 1 und 2 werden 18,25 g (0,069 mol) der Verbindung aus dem Beispiel VI in 300 ml dem. Wasser und 200 ml MIBK bei 32,5°C und pH 7,5, mit dem löslichen Anteil von 2 g Maxatase puur (GIST-BROCADES) in 2 Stunden gespalten.

b) Isolierung des Beispiels 3

Der nicht gespaltene Ester wurde bei pH 7,5 mit 3 mal 250 ml MIBK nach HPLC extrahiert.
Ausbeute: 8,15 g (89,5% der Theorie)

c) Isolierung des Beispiels 4

Die gebildete Säure wurde nach dem Einstellen des pH-Wertes mit 18%iger Salzsäure auf pH 1,0 mit 12 mal 250 ml MIBK extrahiert.
Ausbeute: 4,68 g (54,2% der Theorie)
Analytische Daten zu Beispiel 3 und 4

Beispiel 3

$\alpha[D]_{589}$ = +27,8°; c = 1 in Methanol
DC-System IV = 0,76
MS (DCI: m/z = 264 (M+H)

Beispiel 4

$\alpha[D]_{589}$ = -9,8°; c = 1 in Methanol

Beispiele 5 und 6

2-S-Amino-2-[2-(1,3-dithiolano)]essigsäureeethylester

(Beispiel 5) und 2-R-Amino-2-[2-(1,3-dithiolano)]essigsäure (Beispiel 6)

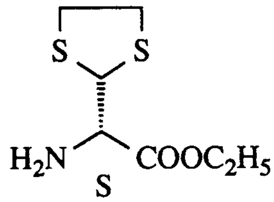

(Beispiel 5)

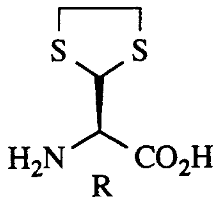

(Beispiel 6)

I a) Enzymatische Spaltung des R,S-Diethylesters

0,97 g (4,7 mmol) 2-R,S-Amino-2-[2-(1,3-dithiolano)]essigsäureesthylester [M.P. Mertes, A.A. Ramsey, J. Med. Chem. 12, 342 (1969)] werden in 25 ml dem. Wasser und 15 ml Methylisobutylketon bei pH 7,8 und 37°C mit dem löslichen Anteil von 500 mg Maxatase puur (GIST-BROCADES, Delft, NL) gespalten. Der pH-Wert wird mit 0,1 N NaOH nachgestellt Nach 5 Stunden ist die Reaktion beendet, die Phasen werden getrennt.

I b) Isolierung des Beispiels 5

Die Wasserphase wird 3 mal mit je 50 ml Methylisobutylketon bei pH 7,8 extrahiert und am Rotationsverdampfer zur Trockene eingeengt.
Ausbeute: 0,164 g

I c) Isolierung des Beispiels 6

Die erhaltene Säure wird nach ansäuern mit 18%iger Salzsäure bei pH 1,5 zur Trockene eingeengt. Es wurde mit 95% Aceton/5% Wasser extrahiert und zur Trockne eingeengt.
Ausbeute: 0,76 g

Weitere Darstellungsmöglichkeiten des Beispiels 6

2-R-Amino-2-[2-(1,3-dithiolano)]essigsäure

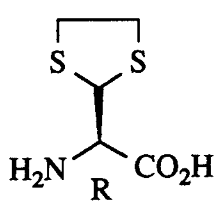

II Esterspaltung der Verbindung aus dem Beispiel 12

260 mg 2-R-Amino-2-[2-(1,3-dithiolano)]essigsäuremethylester Hydrochlorid (Beispiel 12) werden in 30 ml Wasser gelöst und mit 10 mg Maxatase puur (Gist-Brocades) bei 37°C und einem pH von 7,8 gespalten. Nach 5 Stunden wird zur Trockene eingeengt.
Ausbeute: 298 mg

III Deacetylierung des Beispiels 2 mit Acylase

0,1 g der Verbindung aus Beispiel 2 werden in 10 ml 0,1 M Phosphatpuffer pH 7,5 suspendiert und mit 0,1 g Acylase 1 Fa. Serva Nr. 10720 versetzt. Man schüttelt 24 Stunden bei 37°C und analysiert dann mittels DC.

DC-Analytische Daten:
DC-System V: Beispiel 2: $R_f$ = 0,6; Ninhydrin negativ, Jod-positiv
DC-System V: Beispiel 6: $R_f$ = 0,37, Ninhydrin positiv, Jod-positiv
Abreaktion > 95% zu Beispiel 6

IV Enzymatische Spaltung der Acetyl-R,S-Säuren (Beispiel III)

0,1 g der Verbindung aus Beispiel III werden in 10ml 0,1 M Phosphatpuffer pH 7,5 suspendiert und mit 0,1 g Acylase 1 Fa. Serva Nr. 10720 versetzt. Man schüttelt 24 Stunden bei 37°C und analysiert dann mittels DC. Es zeigt eine Abreaktion des Ansatzes etwa zur Hälfte.

DC-analytische Daten:
DC-System V: Beispiel III: $R_f$ = 0,6; Ninhydrin negativ, Jod positiv
DC-System V: Beispiel 6: $R_f$ = 0,37; Ninhydrin positiv, Jod positiv

V Hydrolyse des Beispieles 2

32,2 g (145 mol) N-Acetyl-2-R-[2-(1,3-dithiolano)]essigsäure (Beispiel 2) werden in 600 ml 6 N HCl gelöst und 1 h unter Rückfluß gerührt. Der Ansatz wird heiß vom unlöslichen Rückstand abfiltriert und das Filtrat zur Trockene eingeengt. Der Rückstand wird mit Diethylether verrührt, abgesaugt und über KOH getrocknet. Ausbeute: 30.65 g (98% der Theorie)
$\alpha[D]^{20°}/_{589}$ = -22,08; c = 1 in Methanol
MS-DCI: m/z = 180 (M+H)
$^1$H-NMR (DMSO, 250 MHz): $\delta$ = 3,30 (m, 4H); 4,18 (d, 1H); 5,09 (d, 1H); 8,60 (breit, 3H).
DC-System V: $R_f$ = 0,14

Die Verbindung des Beispiels 6 (V) läßt sich durch Entschwefeln mit Raney-Nickel in das natürliche L-Alanin überführen. Die GC-Analyse ergibt eine optische Reinheit von 89,3% L.

Beispiel 7 und 8

N-tert.-Butoxycarbonyl-2-S-amino-2-[2-(1,3-dithiolano)]essigsäureethylester (Beispiel 7) und N-tert.Butyloxycarbonyl-2-R-amino-2-[2-(1,3-dithiolano)]-essigsäure (Beispiel 8)

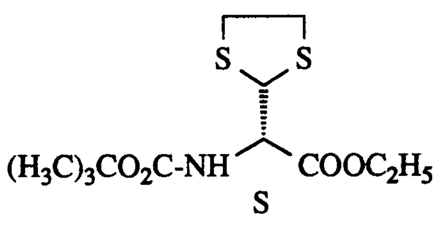

(Beispiel 7)

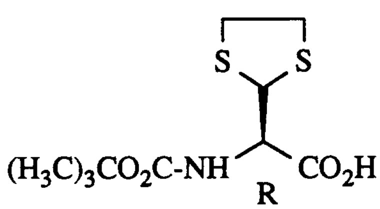

(Beispiel 8)

I a) Enzymatische Spaltung

0,95 g (3,1 mmol) der Verbindung aus Beispiel I werden in 40 ml dem. Wasser und 10 ml MIBK bei pH 7,8 und 37°C mit dem löslichen Anteil von 400 mg Maxatase puur gespalten. Der pH wird mt 0,1 N NaOH

nachgestellt. Nach 70 min ist die Reaktion beendet.

I b) Isolierung des Beispiels 7

Der nicht gespaltene Ester wird mit 3 mal 50 ml MIBK extrahiert und am Rotationsverdampfer zur Trockne eingeengt.
Ausbeute: 0,49 g
$\alpha[D]_{589}$ = +37,1°; c = 1 in Methanol

I c) Isolierung des Beispiels 8 Die Säure wird nach ansäuern mit 18%iger Salzsäure auf pH 1,5 mit 3 mal 50 ml MIBK extrahiert und am Rotationsverdampfer zur Trockene eingeengt.
Ausbeute: 0,41 g
$\alpha[D]_{589}$ = -18,3°; c = 1 in Methanol

II Weitere Darstellung des Beispiels 8

9,27 g (43 mmol) der Verbindung aus Beispiel 6 (V) werden in 112 ml Wasser und 225 ml Dioxan gelöst. Man gibt 5 g (125 mmol) Natriumhydroxidplätzchen dazu und versetzt den Ansatz unter Rühren und Kühlen im Eisbad mit 23,5 g (108 mmol) Di-tert.-Butylcarbonat. Die Mischung wird über Nacht bei Raumtemperatur gerührt und vom Dioxan abrotiert. Man verdünnt mit 300 ml Wasser und extrahiert die basische Wasserphase mit 3 mal 300 ml Diethylether Die Wasserphase wird mit 165 ml 1 N HCl auf pH 3 gestellt und mit 3 mal 300 ml Dichlormethan extrahiert. Die vereinigte organische Phase wird mit gesättigter Natriumchloridlösung gewaschen, über Natriumsulfat getrocknet und eingeengt.
Ausbeute: 11,4 g (95% der Theorie)
DC-System IV: $R_f$ = 0,55
MS-DCI: m/z = 280 (M+H); m/z = 297 ($M+NH_4$)
$^1$H-NMR (DMSO, 250 MHz): $\delta$ = 1,39 (s, 9H); 3,17 (m, 4H); 4,02 (dd, 1H); 4,72 (d, 1H); 7,28 (d, 1H); 12,80 (breit, 1H).
$\alpha[D]^{20}/_{589}$ = -17,71°; c = 1 in Methanol

Beispiel 9

N-Acetyl-S-amino-2-[2-(1,3-dithiolano)essigsäure

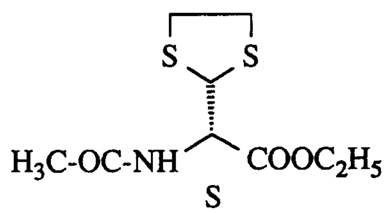

0,1 g der Verbindung aus Beispiel III werden in 10 ml 0,1 M Phosphatpuffer pH 7,5 suspendiert und mit 0,1 g Acylase 1 Fa. Serva Nr. 10720 versetzt. Man schüttelt 24 Stunden bei 37°C und analysiert dann mittels DC. Es zeigt eine Abreaktion des Ansatzes etwa zur Hälfte.

DC-analytische Daten:
DC-System V: Beispiel III: $R_f$ = 0,6; Ninhydrin negativ, Jod positiv
(DC-System V: Beispiel 6: $R_f$ = 0,37; Ninhydrin positiv, Jod positiv)
DC-System V: Beispiel 9: $R_f$ = 0,6; Ninhydrin negativ, Jod positiv

Beispiel 10 und 11

2-R-Amino-2-[2-(1,3-dithiolano)]essigsäureethylester (Beipsiel 10) und
2-S-Amino-2-[2-(1,3-dithiolano)essigsäureethylester(Beispiel 11)

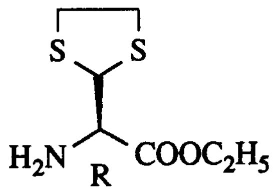

(Beispiel 10)

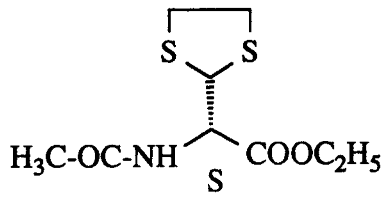

(Beispiel 11)

0,1 g der Verbindung aus Beispiel II werden in 10 ml 0,1 M Phosphatpuffer pH 7,5 suspendiert und mit 0,1 g Acylase 1 Fa. Serva Nr. 10720 versetzt. Man schüttelt 24 Stunden bei 37° C und analysiert dann mittels DC. Bei der Umsetzung des Esters entsteht laut DC zusätzlich etwas der Verbindung des Beispiels 6. DC-analytische Daten:
DC-System V: Beispiel 10: $R_f$ = 0,65, Ninhydrin positiv, Jod positiv
DC-System V: Beispiel 11: $R_f$ = 0,80; Ninhydridn negativ, Jod positiv

Beispiel 12

2-R-Amino-2-[2-(1,3-dithiolano)]essigsäuremethylester Hydrochlorid

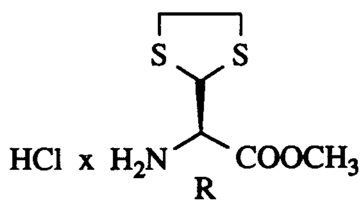

22,6 g (102 mmol) N-Acetyl-2-R-amino-2-[2-(1,3-dithiolano)]essigsäure (Beispiel 2) werden in 500 ml Methanol gelöst und unter Rühren mit 18,2 ml (0,25 mol) Thionylchlorid versetzt. Der Ansatz wird 3 Tage am Rückfluß erhitzt und eingeengt. De Rückstand wird in 250 ml 1 N Salzsäure aufgenommen und 3 mal mit je 200 ml Dichlormethan ausgeschüttelt. Die wäßrige Phase wird mit gesättigter Natriumbicarbonatlösung auf pH 7,5 - 8 gestellt und 3 mal mit je 200 ml Dichlormethan extrahiert. Die vereinigte organische Phase wird über Natriumsulfat getrocknet und eingeengt. Der Rückstand wird in Diethylether aufgenommen und mit 4 N Salzsäure in Dioxan versetzt. Das ausgefallene Hydrochlorid wird abgesaugt, mit Diethylether verrieben, nochmals abgesaugt und über KOH getrocknet.
Ausbeute: 9,95 g (42,6% der Theorie)
DC-System III: $R_f$ = 0,88
MS-EI: m/z = 193 ($M^+$); m/z 134; m/z 106 (Basispeak)
$\alpha[D]_{589}^{20°C}$ = -24,67; c = 1 Methanol

Beispiel 13

2-S-Amino-2-[2-(1,3-dithiolano)]essigsäure Hydrochlorid

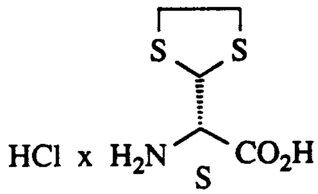

19,8 g (79,4 mmol) N-Acetyl-2-S-[2-(1,3-dithiolano)]essigsäureethylester (Beispiel 1) werden in 400 ml 6 N HCl unter Rückfluß gerührt. Der Ansatz wird heiß vom unlöslichen, schwarzen Rückstand abfiltriert und das Filtrat wird zur Trockene eingeengt. Der Rückstand wird mit Diethylether verrührt, abgesaugt und über KOH getrocknet.
Ausbeute: 16,27 g (95% der Theorie)
$\alpha[D]^{20°C}_{589}$ = +30,29; c = 1 in Methanol
Die Verbindung des Beispiels 13 läßt sich durch Entschwefeln mit Raney-Nickel in das unnatürliche D-Alanin überführen.

Die in Tabelle 2 aufgeführten Beispiele werden analog hergestellt.

Abkürzungen in Tabelle 2:

| | |
|---|---|
| $Ac_2O$ | Essigsäureanhydrid |
| TEA | Triethylamin |
| FMOC-O-Su | Fluorenylmethyloxycarbonyl-O-N-succinimid |
| Z-O-Su | Benzyloxycarbonyl-O-N-succinimid |
| DCC | Dicyclohexylcarbodiimid |
| DMAP | Dimethylaminopyridin |

Tabelle 2:

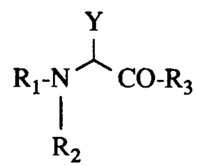

| Bsp.-Nr. | $R^1$ | $R^2$ | $R^3$ | Y | Ausgangs-verbindung | Reagenz | Ausbeute (% d. Th.) | DC-System | F(°C) |
|---|---|---|---|---|---|---|---|---|---|
| 14 | H | H | $-OCH_3$ | 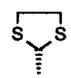 (S), D | 13 | $SOCl_2$/MeOH analog Bsp. 12 | 78,3 | III, 0,88 α[D] (589/20°C)= +23,03 c= 1 Methanol | |
| 15 | -CO-$CH_3$ | H | $-OC_2H_5$ | 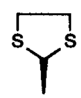 R | 2 | 1.) $SOCl_2$/EtOH 2.) $Ac_2O$/TEA analog Bsp. 12 und I | 55,4 | | 122 |
| 16 | -Boc | H | -OH | 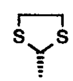 S | 13 | Dioxan/NaOH/ Di-tert.Butyl-carbonat analog Bsp. 8 (II) | 95,6 | IV, 0,55 α[D] (589/20°C)= +20,16 c = 1 Methanol | |

Fortsetzung Tabelle 2:

| Bsp.-Nr. | $R^1$ | $R^2$ | $R^3$ | Y | Ausgangs-verbindung | Reagenz | Ausbeute (% d. Th.) | DC-System | F(°C) |
|---|---|---|---|---|---|---|---|---|---|
| 17 | H | H | -OH | 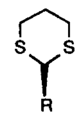 | 4 | Analog Beispiel 6 (V) | | III = 0,05<br>V = 0,35 | |
| 18 | H | H | -OH | 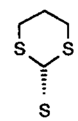 | 3 | Analog Beispiel 13 | | III = 0,05<br>V = 0,35 | |
| 19 | H | H | $-OCH_3$ | 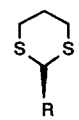 | 4 | Analog Beispiel 12 | | | |
| 20 | H | H | $-OCH_3$ | 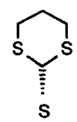 | 18 | Analog Beispiel 14 | | | |
| 21 | -Boc | H | -OH | 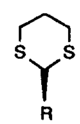 | 19 | Analog Beispiel 8 (II) | | I = 0,19<br>II = 0,12<br>IV = 0,55<br>V = 0,86 | |
| 22 | -Boc | H | -OH | 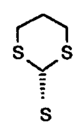 | 18 | Analog Beispiel 16 | | I = 0,19<br>II = 0,12<br>IV = 0,55<br>V = 0,86 | |

Fortsetzung Tabelle 2:

| Bsp.-Nr. | $R^1$ | $R^2$ | $R^3$ | Y | Ausgangs-verbindung | Reagenz | DC-System | (-)FAB-MS |
|---|---|---|---|---|---|---|---|---|
| 23 | Fmoc | H | -OH | 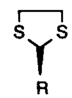 | 6 | Fmoc-O-Su | IV = 0,59<br>α[D] (589/20°C)=<br>-18,22<br>c = 1 DMSO | m/z 400<br>(M-H) |
| 24 | Z | H | -OH | 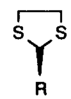 | 6 | Z-O-Su | IV = 0,60<br>α[D] (589/20°C)=<br>-17,68<br>c = 1 DMSO | m/z 312<br>(M-H) |
| 25 | Alloc | H | -OH | 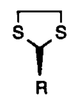 | 6 | | | |
| 26 | H | H | -OBzl | 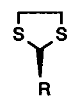 | 6 | Bzl-OH/<br>DCC/DMAP | | |
| 27 | H | H | $-O-C(CH_3)_3$ | 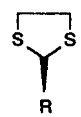 | 6 | 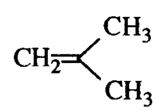 /<br>$H_2SO_4$ | | |
| 28 | H | H | $-OCH_2CH=CH_2$ | 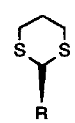 | 6 | | | |

Fortsetzung Tabelle 2:

| Bsp.-Nr. | $R^1$ | $R^2$ | $R^3$ | Y | Ausgangs-verbindung | Reagenz |
|---|---|---|---|---|---|---|
| 29 | H | H | $-OCH(CH_3)_2$ | 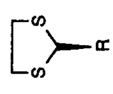 | 6 | $HOCH(CH_3)_2$/ $SOCl_2$ |
| 30 | $C_8H_{17}CO$ | H | -OH | 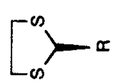 | 6 | $C_8H_{17}COCl$/ TEA |
| 31 | H | H | $-OC_8H_{17}$ | 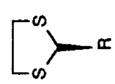 | 6 | $C_8H_{17}OH$/ DCC/DMAP |

## Patentansprüche

1. Optisch reine Verbindungen der allgemeinen Formel I

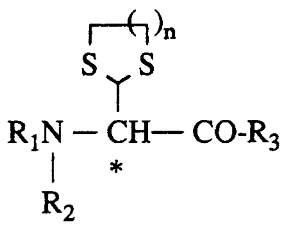 (I)

in welcher

n für die Zahl 1 oder 2 steht,

$R^1$ für Wasserstoff, geradkettiges oder verzweigtes Alkyl mit bis zu 8 Kohlenstoffatomen, Benzyl oder für eine Gruppe der Formel $-COR^4$ steht,

worin

$R^4$ geradkettiges oder verzweigtes Alkyl mit bis zu 10 Kohlenstoffatomen oder Aryl mit 6 bis 10 Kohlenstoffatomen bedeutet, oder

für eine typische Aminoschutzgruppe steht,

$R^2$ für Wasserstoff, geradkettiges oder verzweigtes Alkyl mit bis zu 8 Kohlenstoffatomen oder Benzyl steht, oder

$R^1$ und $R^2$ gemeinsam mit dem Stickstoffatom einen Morpholinring oder einen Phthalimidorest bilden,

$R^3$ für Hydroxy, geradkettiges oder verzweigtes, gegebenenfalls durch Vinyl substituiertes Alkoxy mit bis zu 10 Kohlenstoffatomen, Benzyloxy oder für die Gruppe $-NR^5R^6$ steht, worin

$R^5$ und $R^6$ gleich oder verschieden sind und Wasserstoff, geradkettiges oder verzweigtes Alkyl mit bis zu 6 Kohlenstoffatomen oder Phenyl bedeuten,

und das mit * gekennzeichnete C-Atom entweder in der S- oder R-Form vorliegt.

**2.** Verbindungen der allgemeinen Formel I gemäß Anspruch 1, in welcher

n für eine Zahl 1 oder 2 steht,

$R^1$ für Wasserstoff, geradkettiges oder verzweigtes Alkyl mit bis zu 6 Kohlenstoffatomen oder Benzyl steht, oder
für eine Gruppe der Formel $-COR^4$ steht,

worin

$R^4$ geradkettiges oder verzweigtes Alkyl mit bis zu 8 Kohlenstoffatomen oder Phenyl bedeutet, oder

für tert.Butoxycarbonyl (Boc), Benzyloxycarbonyl (Z), Ethoxycarbonyl (EtOC), Allyloxycarbonyl (AllOC), Fluorenyl-9-methoxycarbonyl (Fmoc) oder Methoxycarbonyl (MetOC) steht,

$R^2$ für Wasserstoff, geradkettiges oder verzweigtes Alkyl mit bis zu 6 Kohlenstoffatomen oder Benzyl steht, oder

$R^1$ und $R^2$ gemeinsam mit dem Stickstoffatom den Phthalimidorest bilden,

$R^3$ für Hydroxy, geradkettiges oder verzweigtes, gegebenenfalls durch Vinyl substituiertes Alkoxy mit bis zu 8 Kohlenstoffatomen, Benzyloxy oder für die Gruppe $-NR^5R^6$ steht,

worin

$R^5$ und $R^6$ gleich oder verschieden sind und Wasserstoff, geradkettiges oder verzweigtes Alkyl mit bis zu 4 Kohlenstoffatomen oder Phenyl bedeuten,

und das mit * gekennzeichnete C-Atom entweder in der S- oder R-Form vorliegt.

**3.** Verbindungen der allgemeinen Formel I gemäß Anspruch 1, in welcher

n für die Zahl 1 oder 2 steht,

$R^1$ für Wasserstoff, geradkettiges oder verzweigtes Alkyl mit bis zu 4 Kohlenstoffatomen oder Benzyl steht, oder für eine Gruppe der Formel -CO-$R^4$ steht,

worin

$R^4$ geradkettiges oder verzweigtes Alkyl mit bis zu 8 Kohlenstoffatomen oder Phenyl bedeutet, oder

für tert.-Butoxycarbonyl (BOC), Benzyloxycarbonyl(Z), Allyloxycarbonyl (AllOC), Ethoxycarbonyl (EtOC) oder Fluorenyl-9-methoxycarbonyl (Fmoc) steht,

$R^2$ für Wasserstoff, geradkettiges oder verzweigtes Alkyl mit bis zu 4 Kohlenstoffatomen oder Benzyl steht, oder

$R^1$ und $R^2$ gemeinsam mit dem Stickstoffatom den Phthalimidorest bilden,

$R^3$ für Hydroxy, geradkettiges oder verzweigtes, gegebenenfalls durch Vinyl substituiertes Alkoxy mit bis zu 8 Kohlenstoffatomen, Benzyloxy oder für die Gruppe -$NR^5R^6$ steht,

worin

$R^5$ und $R^6$ gleich oder verschieden sind und Wasserstoff, Methyl, Ethyl, Propyl, Isopropyl oder Phenyl bedeuten.

**4.** Verfahren zur Herstellung von Verbindungen der allgemeinen Formel I

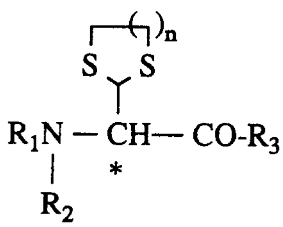

(I)

in welcher

n für die Zahl 1 oder 2 steht,

$R^1$ für Wasserstoff, geradkettiges oder verzweigtes Alkyl mit bis zu 8 Kohlenstoffatomen, Benzyl oder für eine Gruppe der Formel -$COR^4$ steht,

worin

$R^4$ geradkettiges oder verzweigtes Alkyl mit bis zu 10 Kohlenstoffatomen oder Aryl mit 6 bis 10 Kohlenstoffatomen bedeutet, oder

für eine typische Aminoschutzgruppe steht,

$R^2$ für Wasserstoff, geradkettiges oder verzweigtes Alkyl mit bis zu 8 Kohlenstoffatomen oder Benzyl steht, oder

$R^1$ und $R^2$ gemeinsam mit dem Stickstoffatom einen Morpholinring oder einen Phthalimidorest bilden,

$R^3$ für Hydroxy, geradkettiges oder verzweigtes, gegebenenfalls durch Vinyl substituiertes Alkoxy mit bis zu 10 Kohlenstoffatomen, Benzyloxy oder für die Gruppe -$NR^5R^6$ steht,

worin

$R^5$ und $R^6$ gleich oder verschieden sind und Wasserstoff, geradkettiges oder verzweigtes Alkyl mit bis zu 6 Kohlenstoffatomen oder Phenyl bedeuten,

und das mit * gekennzeichnete C-Atom entweder in der S- oder R-Form vorliegt, dadurch gekennzeichnet, daß man racemische Verbindungen der allgemeinen Formel II

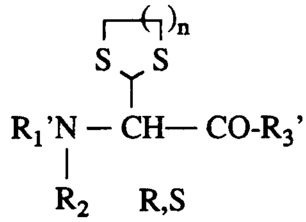

(II)

in welcher

n und $R^2$ die oben angegebene Bedeutung haben,

$R^{1'}$ und $R^{3'}$ die oben angegebenen Bedeutungen von $R^1$ und $R^3$ haben und mit diesen gleich oder verschieden sind, aber $R^{1'}$ nicht für Wasserstoff und $R^{3'}$ nicht für Hydroxy stehen darf,

unter Einwirkung von spezifisch spaltenden trägergebundenen und/oder freien proteolytischen Enzymen, in einem zweiphasigen Medium, bestehend aus Wasser und einem mit Wasser nicht mischbaren Lösemittel, in die optisch reinen R-Verbindungen der Formeln (III) oder (IV)

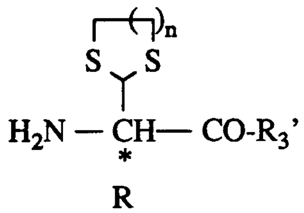

(III) oder

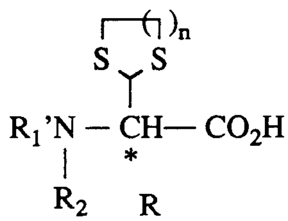

(IV)

in welchen

n, $R^1$, $R^{1'}$, $R^2$ und $R^{3'}$ die oben angegebene Bedeutung haben,

überführt,

von den ebenfalls anfallenden optisch reinen S-Verbindungen der Formel (IIa)

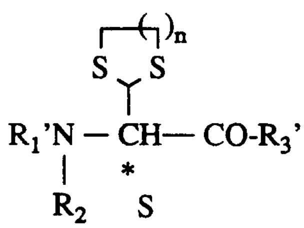

(IIa)

in welcher

$R^{1'}$, $R^2$ und $R^{3'}$ die oben angegebene Bedeutung haben,

nach üblicher Methode abtrennt, wobei diese nach üblicher chemischer Racemisierung dem enzymatischen Prozeß erneut zugeführt werden können, und

in einem letzten Schritt gegebenenfalls die Substituenten $R^{1'}$ und/oder $R^{3'}$ in den Verbindungen der allgemeinen Formeln (IIa), (III) und (IV) nach üblicher chemischer Methode in die Substituenten $R^1$ und $R^3$ überführt.

**5.** Verfahren gemäß Anspruch 4, dadurch gekennzeichnet, daß man die enzymatische Spaltung in einem Temperaturbereich von 20 bis 40 ° C und in einem pH-Bereich von 6 bis 8 durchführt.

**6.** Verwendung der Verbindungen der allgemeinen Formel I gemäß Anspruch 1 zur Herstellung von Peptiden.

**7.** Verwendung von Verbindungen der allgemeinen Formel I gemäß Anspruch 1 zur Herstellung von Peptiden mit renininhibitorischer Wirkung.

Europäisches Patentamt

# EUROPÄISCHER RECHERCHENBERICHT

Nummer der Anmeldung

EP 91 12 0028

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| X<br><br>D | EP-A-0 022 206 (BAYER)<br>* Seite 1; Ansprüche 1,3 *<br>& US-A-4 389 489<br>--- | 1,4 | C07D339/06<br>C07D339/08<br>C12P41/00<br>C12P13/04 |
| D,A | JOURNAL OF MEDICINAL CHEMISTRY,<br>Bd. 12, Nr. 2, Mai 1969, Washington, DC, US,<br>Seiten 342 - 343;<br>M.P. MERTES ET AL.: 'omega-Dithiolano amino acids'<br>* Verbindungen 1, 6, 7 *<br>--- | 1 | |
| P,A | EP-A-0 403 828 (BAYER)<br>* Seite 17 - Seite 18; Beispiele 5,10-15,60-63 *<br>----- | 1-3,6,7 | |

| RECHERCHIERTE SACHGEBIETE (Int. Cl.5) |
|---|
| C07D |

Der vorliegende Recherchenbericht wurde für alle Patentansprüche erstellt

| Recherchenort | Abschlußdatum der Recherche | Prüfer |
|---|---|---|
| DEN HAAG | 14 FEBRUAR 1992 | RUSSELL F. ENGLISH |

**KATEGORIE DER GENANNTEN DOKUMENTE**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument